(19) Europäisches Patentamt
European Patent Office
Office européen des brevets

(11) Numéro de publication: **0 001 534**
**B1**

(12) **FASCICULE DE BREVET EUROPEEN**

(45) Date de publication du fascicule de brevet: **12.08.81**

(21) Numéro de dépôt: **78400130.7**

(22) Date de dépôt: **06.10.78**

(51) Int. Cl.³: **C 07 D 207/34,**
A 61 K 31/40 //C07D403/06,
C07D405/06, C07D409/06

(54) **Nouveaux dérivés du pyrrole, leur procédé de préparation et leurs applications en thérapeutique.**

(30) Priorité: **10.10.77 GB 4210577**
**18.05.78 GB 4210577**

(43) Date de publication de la demande:
**18.04.79 Bulletin 79/8**

(45) Mention de la délivrance du brevet:
**12.08.81 Bulletin 81/32**

(84) Etats Contractants Désignés:
**DE NL SE**

(56) Documents cités:
DE - A - 2 511 256
DE - A - 2 524 299
DE - A - 2 628 475
FR - A - 2 313 364
US - A - 3 752 826
US - A - 3 952 012

(73) Titulaire: **Albert ROLLAND S.A. Société dite**
**49, Rue St-André-des-Arts**
**F-75006 Paris (FR)**

(72) Inventeur: **Laforest, Jacqueline**
**106, Boulevard de la Libération**
**F-94300 Vincennes (FR)**
Inventeur: **Bonnet, Jacqueline**
**10 Rue Salignat**
**03200 Vichy (FR)**
Inventeur: **Bessin, Pierre**
**1, Allée Bossuet**
**91380 Chilly-Mazarin (FR)**

(74) Mandataire: **Lavoix, Jean et al,**
**c/o Cabinet Lavoix 2, Place D'Estienne D'Orves**
**F-75441 Paris Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 001 534 B1

Courier Press, Leamington Spa, England.

# Nouveaux dérivés du pyrrole, leur procédé de préparation et leurs applications en thérapeutique

La présente invention concerne de nouveaux dérivés du pyrrole, leur procédé de préparation ainsi que leur application comme médicaments pour traiter, en particulier, l'hyperuricémie.

Le noyau pyrrolique est la structure de base des porphyrines et certains dérivés du pyrrole sont connus comme ayant un intérêt thérapeutique, notamment la tolmétine (anti-inflammatoire) de formule

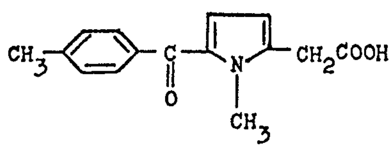

et plus généralement des acides 5 - aroyl - pyrrole - alcanoïques de formule

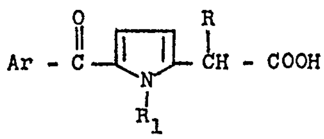

(brevets U.S. 3 752 826 et 3 952 012).

La présente invention a pour objet des dérivés, d'acide pyrrole carboxylique de formule générale I

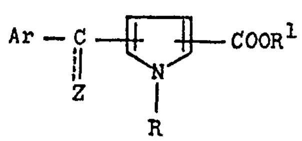

(I)

dans laquelle

$R^1$ représente un atome d'hydrogène ou un groupe alkyle ayant de 1 à 4 atomes de carbone,

R représente un groupe alkyle ayant de 1 à 6 atomes de carbone, un groupe benzyle ou un groupe phényle,

Ar représente un cycle aromatique choisi parmi les groupes phényle substitués ou non, les groupes naphtyle substitués ou non et les radicaux hétérocycliques aromatiques tels que thiényle, furyle ou pyrrolyle, et

>C⩵Z représente un groupe carbonyle >C=O, un groupe de formule >C=NOH ou un groupe alcoolique >CHOH, ainsi que les sels des acides de formule I avec des bases physiologiquement acceptables, et les dérivés fontionnels des composés de formule I qui so transforment par voie métabolique en ces composés.

Les noyaux phényle et naphtyle, considérés ici, peuvent être mono- ou polysubstitués par des radicaux alkyle, alcoxy, halogène, les groupements alkyle et alcoxy pouvant comporter de 1 à 4 atomes de carbone.

Les composés de formule I dans lesquels >C⩵Z est un groupe carbonyle peuvent être préparés par acylation d'un composé de formule

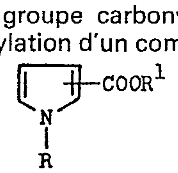

(II)

dans laquelle R et $R^1$ ont les significations données ci-dessus, par un acide de formule Ar COOH ou l'un de ses dérivés réactifs tel qu'ester, chlorure d'acide, anhydride, Ar ayant la même signification que ci-dessus. L'acylation par un acide est de préférence effectuée en présence d'acide polyphosphorique ou d'un réactif connu comme équivalent. L'acylation par un chlorure d'acide, anhydride ou ester est effectuée dans les conditions habituelles d'une réaction de Friedel et Crafts. Plus particulièrement, on peut faire réagir un chlorure d'acide ArCOCl avec un composé de formule II en solution dans un solvant tel que le dichloréthane ou le chlorure de méthylène, à une température inférieure à 30°C, et en utilisant au moins un équivalent molaire de chlorure métallique, tel $AlCl_3$, $SnCl_4$, comme catalyseur, cette opération peut être suivie de l'hydrolyse de l'ester obtenu suivant des procédés connus et par exemple en milieu alcoolique ou hydroalcoolique, en présence d'une base telle qu'un hydroxyde ou carbonate métallique.

Suivant les conditions opératoires et les réactifs en présence, lors de la réaction avec les composés de formule II, l'acylation se fait de façon prédominante en position 4 ou en position 5 du pyrrole. Les températures inférieures à 0°C favorisent l'acylation en position 4 des dérivés d'acide pyrrole-2 carboxylique.

On peut séparer ces isomères par chromatographie en phase liquide ou par recristallisations successives, grâce à leurs différences de solubilité.

Les composés de formule I peuvent également être préparés par réaction de composés de formule I dans laquelle R représente un atome d'hydrogène et $R^1$ et Ar ont la signification donnée précédemment avec un dérivé halogéné RX dans laquelle R est un radical alcoyle de 1 à 6 atomes de carbone ou un radical benzyle et X est un atome d'halogène, en présence d'un accepteur d'acide. Cette réaction est de préférence conduite dans un solvant polaire aprotique tel que le diméthylformamide ou le diméthylsulfoxyde, par exemple, et en présence d'un hydroxyde alcalin, avec si nécessaire hydrolyse ultérieure de l'ester.

Les composés de formule I dans lesquels Z est un groupe hydroxyimino ou représente un groupe alcoolique secondaire peuvent être préparés à partir des cétones de formule I (Z = O) par des procédés classiques. Ainsi, les oximes peuvent être obtenues par action du chlorhydrate d'hydroxylamine sur les acides cétoniques de formule I ou leurs esters, en présence d'une base telle que la pyridine, qui peut être utilisée comme solvant, opération suivie, si nécessaire, de l'hydrolyse de l'ester. Les alcools peuvent être préparés par réduction chimique des fonctions cétones, par exemple

par le borohydrure de sodium en solution hydroalcoolique.

La salification des acides de formule I par des bases physiologiquement acceptables minérales ou organiques, et telles qu'hydroxydes, carbonates métalliques ou amines, peut être effectuée de manière classique par action de l'acide sur la base dans un solvant convenable.

Les exemples suivants illustrent la préparation des composés de formule I. Dans ces exemples, les composés préparés suivants ont été soumis à une étude analytique, qui a confirmé leur structure et a montré que certains composés présentent diverses formes cristallines; les points de fusion mentionnés, dans ce qui suit, sont les points de fusion instantanés.

Exemple 1

Acide méthyl-1 (me hoxy-4-naphtoyl-1)-4 pyrrole-2 carboxylique

a) On dissout dans 400 ml de dichloro-1,2 éthane 35 g de chlorure d'acide méthoxy-4 naphtoïque, (préparé par action du pentachlorure de phosphore sur l'acid de F = 250°C), puis 22 g de méthyl-1 pyrrole-2 carboxylate de méthyle. Sous agitation, vers −20°C, on introduit lentement dans le milieu 25 g de chlorure d'aluminium anhydre. Au bout d'une heure, on verse sur 1 kg de glace pilée. La phase organique est décantée et la phase aqueuse extraite par 500 ml de chloroforme. Après dessication, les solvants sont évaporés; les 28 g de résidu solide sont lavés par 200 ml d'éther isopropylique.

On obtient ainsi avec 50% de rendement le méthyl-1 (méthoxy-4 naphtyl-1)-4 pyrrole-2 carboxylate de méthyle; F = 162°C.

b) 24 g de l'ester méthylique obtenu en (a) sont dissous dans 120 ml d'éthanol et 120 ml de solution aqueuse 5N d'hydroxyde de sodium; la solution est portée une heure à sa température de reflux. Après refroidissement, on acidifie par addition d'une solution aqueuse concentrée d'acide chlorhydrique, puis extrait dans la méthyléthylcétone 20 g d'acide final; F = 249°C.

Exemple 2

Acide méthyl-1 (chloro-3 benzoyl)-4 pyrrole-2 carboxylique

a) On introduit dans 100 ml de dichloro - 1,2 éthane 5 g de chlorure de chloro - 3 benzoyle, puis 8,4 g de chlorure d'aluminium. Le mélange est refroidi à 5°C et 4 g de méthyl-1 pyrrole-2 carboxylate de méthyle en solution dans 15 ml de dichloro - 1,2 éthane sont introduits. Après 1/2 heure d'agitation à cette température, puis 1 heure à température ambiante, la solution est hydrolysée sur glace, la phase organique décantée et le solvant évaporé. Après recristallisation du solide résiduel dans le méthanol, on obtient 7,5 g de méthyl- (chloro-3-benzoyl)-4 pyrrole-2 carboxylate de méthyle; F = 126°C.

En variante, on dissout 10,6 g d'anhydride de l'acide chloro - 3 benzoïque dans 120 ml de nitrométhane, on ajoute 14,5 g de chlorure d'aluminium, puis lentement la méthyl-1 pyrrole-2 carboxylate de méthyle. Au bout de 3 heures à température ambiante, on traite le mélange de manière usuelle et on obtient l'ester avec un rendement de 65%.

b) 5 g de l'ester obtenu en (a) sont hydrolysés, en solution dans 60 ml d'éthanol aqueux à 50%, par action de 2 g d'hydroxyde de potassium. La solution est maintenue 6 heures à sa température de reflux, puis acidifiée par addition d'acide chlorhydrique. 4,7 g d'acide précipitent qui sont recristallisés dans la butanone-2 ou dans un mélange (75/50) d'acétone et eau; F = 214°C

c) Par action de 1,25 g d'hydroxyde de potassium sur 5 g de l'acide obtenu en (b) en solution dans 50 ml d'éthanol, on obtient le sel de potassium de cet acide qui fond à 345°C.

d) En variante, on prépare l'acide de la façon suivante: on introduit 26,7 g de chlorure d'aluminium dans une solution de 17,7 g de chlorure de l'acide chlor-3 benzoique dans 300 ml de dichloro-1,2 éthane. Vers 0°C, on ajoute, peu à peu, une solution de 12,5 g d'acide méthyl-1 pyrrole-2 carboxylique dans 200 ml de dichloro-1,2 éthane. Après 3 heures d'agitation à température ambiante, le mélange est versé sur glace. Le précipité formé est recristallisé dans 250 ml de butanone-2 pour donner 20 g d'acide pur; F = 214°C.

Exemple 3

Acide méthyl-1 ($\alpha$-hydroxy chloro-3 benzyl)-4 pyrrole-2 carboxylique

On dissout 5 g d'acide méthyl-1 (chloro-3 benzoyl)-4 pyrrole-2 carboxylique obtenu à l'exemple 2 dans 40 ml d'éthanol aqueux (96%). On introduit 4 ml de solution aqueuse d'hydroxyde de sodium (d = 1,33) et 0,7 g de borohydrure de sodium. Après 24 heures à température ambiante, la solution est acidifiée par addition d'acide acétique, 3 g d'acide hydroxylé précipitent. F = 180°C.

Exemple 4

Acide propyl-1 (naphtoyl-1)-4 pyrrole-2 carboxylique

a) Dans une solution de 9 g d'hydroxyde de potassium, dans 25 ml de diméthylsulfoxyde, on introduit 5 g de pyrrole-2 carboxylate de méthyle et 10 g de bromo-1 propane. Après 1 heure d'agitation, on verse sur 100 g de glace pilée, puis extrait la solution à l'éther éthylique. Après dessication, on évapore le solvant sous pression réduite pour obtenir 5 g de propyl-1 pyrrole-2 carboxylate de méthyle brut.

b) A 0°C, dans 75 ml d'une solution dans le dichloro-1,2 éthane de 5 g de l'ester obtenu en (a) et de 5,4 g de chlorure de naphtoyle-1, on introduit 3,9 g de chlorure d'aluminium. Après 1/2 heure d'agitation à 0°C, puis 1 heure à température ambiante, on hydrolyse sur glace. La phase organique est décantée et le solvant évaporé; on obtient 5 g d'huile. Cette huile est

mise en solution dans 150 ml d'éthanol aqueux (50%) contenant 1 g d'hydroxyde de potassium. Après quelques heures de reflux, on élimine l'éthanol sous pression réduite et acidifie la phase aqueuse. Le produit final est extrait dans le chloroforme. Après recristallisation dans le benzène, on obtient 3,5 g d'acide fondant à 145°C; une autre forme cristalline de cet acide fond à 175°C.

Exemple 5

Acide méthyl-1 (naphtoyl-1)-4 pyrrole-2 carboxylique

On dissout 11,2 g de méthyl-1 pyrrole-2 carboxylate de méthyle et 15 g de chlorure de naphtoyle-1 dans 150 ml de chlorure de méthylène et introduit, sous agitation dans la solution, lentement, 11,3 g de chlorure d'aluminium. Après 2 heures à la température de reflux du solvant, on verse le mélange sur 100 g de glace pilée et ajoute 10 ml d'acide chlorhydrique concentré; la phase organique décantée est lavée par une solution aqueuse basique, puis concentrée pour donner 20g de résidu huileux qui contient un peur des produits de départ et un mélange de 2 isomères de position: les méthyl-1 naphtoyl-4 et -5 pyrrole-2 carboxylates de méthyle.

Le dérivé acylé en position 4 du pyrrole, qui s'est formé préférentiellement est isolé en tenant compte de sa plus faible solubilité dans le méthanol. Les 20 g d'huile sont introduits dans 100 ml de méthanol au reflux; 11 g de méthyl-1 naphtoyl-4 pyrrole-2 carboxylate de méthyle précipient au refroidissement. F = 124°C. Ils sont hydrolysés par action d'une base en milieu hydroalcoolique, comme décrit dans les exemples précédents, pour donner 9,5 g d'acide. F = 216°C.

Le sel de morpholine de l'acide obtenu ainsi est préparé par action d'une molécule d'acide sur une molécule de morpholine, en solution dans l'éthanol. Il est recristallisé dans l'acétone. F = 155°C.

Exemple 6

Acide méthyl-1 (naphtoyl-1)-5 pyrrole-2 carboxylique

La solution méthanolique obtenue dans l'exemple 5 après filtration du précipité de méthyl-1(naphtoyl-1)-4 pyrrole-2 carboxylate de méthyle est concentrée et l'huile résiduelle est soumise à une chromatographie de partage liquide sur colonne de silice, l'élution étant réalisée avec du dichloro-1,2 éthane; on isole ainsi 3 g de méthyl-1(naphtoyl-1)-5 pyrrole-2 carboxylate de méthyle qui fond à 80°C. Son hydrolyse en milieu hydroalcoolique basique conduit à l'acide de point de fusion 174°C.

Exemple 7

Acide méthyl-1 (naphtyl-1) hydroxy-imino-méthyl)-4 pyrrole-2 carboxylique

On dissout 5 g d'acide méthyl-1(naphtoyl-1)-4 pyrrole-2 carboxylique obtenu à l'exemple 5 et 2 g de chlorhydrate d'hydroxylamine dans 40 ml de pyridine et maintient la solution 7 heures à une température de 60°C. On élimine alors la pyridine sous pression réduite et verse le résidu dans une solution aqueuse 2N d'acide chlorhydrique. Le précipité est isolé. On obtient ainsi avec 85% de rendement un mélange des 2 isomères de l'oxime qui fond à 252°C, en se décomposant.

Exemple 8

Méthyl-1(bromo-2 benzoyl)-4 pyrrole-2 carboxylate de méthyle, méthyl-1(bromo-2 benzoyl)-5 pyrrole-2 carboxylate de méthyle et acides correspondants

On dissout 19 g de méthyl-1 pyrrole-2 carboxylate de méthyle et 30 g de chlorure de bromo-2 benzoyle dans 300 ml de dichloro-1,2 éthane et on ajoute lentement 19 g de chlorure d'aluminium à la solution résultante sous agitation. Au bout de 3 heures à la température ambiante, on verse le mélange sur 500 g de glace pilée. On décante la couche organique et on la concentre après les traitements usuels. On fait cristalliser dans de l'alcool méthylique le solide, obtenu. Ce dernier contient les 2 isomères qui sont séparés par chromatographie liquide de partage (adsorbant:silice, éluent: dichloro-1,2 éthane). L'isomère possédant le groupe benzoyle en position 4 du noyau pyrrole fond à 98°C l'autre à 123°C.

L'hydrolyse de ces 2 esters, par action de l'hydroxyde de potassium dans de l'éthanol aqueux au reflux donne:

l'acide méthyl-1 (bromo-2 benzoyl)-4 pyrrole-2 carboxylique. F = 242°C et l'acide méthyl-1 (bromo-2 benzoyl)-5 pyrrole-2 carboxylique. F = 175°C.

Exemple 9

Méthyl-1(chloro-3 benzoyl)-4(5) pyrrole-3 carboxylates de méthyle et acides correspondants

On ajoute, goutte à goutte, une solution de 6 g d'acide méthyl-1 pyrrole-3 carboxylique dans 100 ml de dichloro-1,2 éthane, à 2°C, à un mélange de 8,75 g de chlorure de chloro-3 benzoyle, de 13,3 g de chlorure d'aluminium et 100 ml de dichloro-1,2 éthane. Après 2 heures a 2°C, puis 5 heures à température ambiante, on introduit de la glace pilée. On estérifie le précipité et les substances organiques dissoutes dans la phase organique, qui contiennent les 2 isomères, pour permettre une séparation par chromatographie liquide.

On dissout 8 g du mélange d'acides ainsi obtenus dans 150 ml de diméthylformamide contenant 8 g d'iodure de méthyle et 7 g de carbonate de potassium et on maintient le reflux pendant 5 heures. On verse de l'eau sur le mélange et on extrait les esters avec de l'oxyde de diéthyle. On effectue une chromatographie de partage sur une colonne de silice en utilisant le dichloro-1,2 éthane comme éluent.

On obtient 2,5 g de méthyl-1 (chloro-3 benzoyl)-4 pyrrole- 3 carboxylate de méthyle

fondant à 86°C et 2,5 g de méthyl-1(chloro-3 benzoyl)-5 pyrrole-3 carboxylate de méthyle fondant à 127°C.

L'hydrolyse de ces esters avec de l'hydroxyde de potassium dans de l'éthanol aqueux donne l'acide méthyl-1 (chloro-3 benzoyl)-4 pyrrole-3 carboxylique. F = 187°C, et l'acide méthyl-1-(chloro-3 benzoyl)-5 pyrrole-3 carboxylique. F = 218°C.

Les composés de formule I, décrits dans les exemples suivants, ont été préparés en appliquant les modes opératoires exposés dans ce qui précède.

| Exemple N° | Formule développée | F°C | Constante physique de l'ester méthylique °C |
|---|---|---|---|
| 10 | 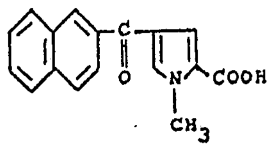 | 262 | F = 138 |
| 11 | 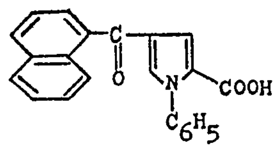 | 214 | |
| 12 | 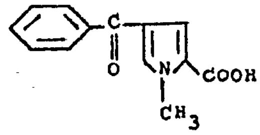 | 252 | F = 120 |
| 13 | 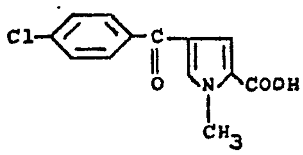 | 216 | F = 160 |
| 14 | 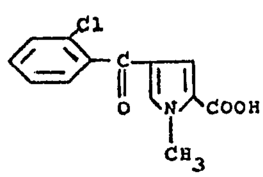 | 238 | F = 102 |
| 15 | 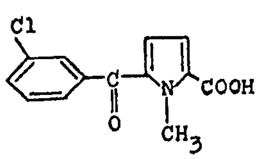 | 200 | |
| 16 | 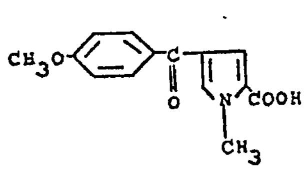 | 204 | F = 135 |

| Exemple N° | Formule développée | F°C | Constante physique de l'ester méthylique °C |
|---|---|---|---|
| 17 | 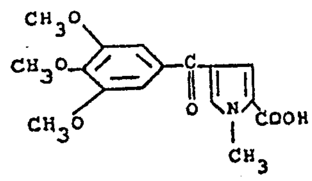 | 195 | F = 149 |
| 18 | 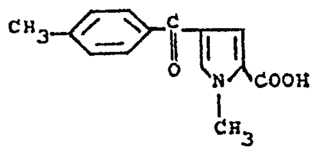 | 217 | F = 148 |
| 19 | 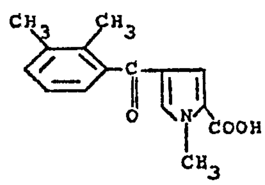 | 226 | F = 97<br>(oxime de l'ester)<br>F = 137 |
| 20 | 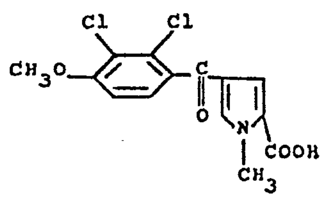 | 275 | F = 190 |
| 21 | 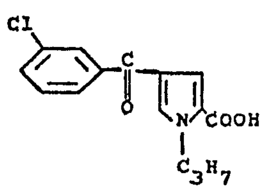 | 191 | |
| 22 | 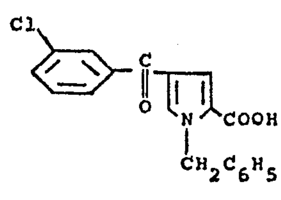 | 140 | |
| 23 | 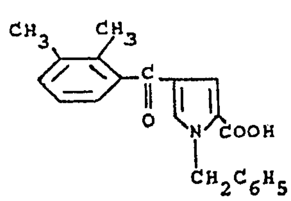 | 173 | |

| Exemple N° | Formule développée | F°C | Constante physique de l'ester méthylique °C |
|---|---|---|---|
| 24 | 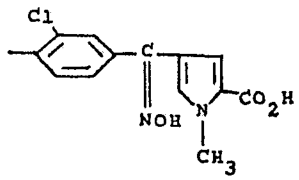 | 230 | |
| 25 | 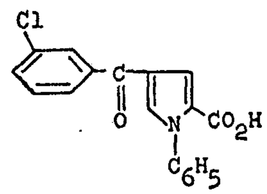 | 185 | F = 106 |
| 26 | 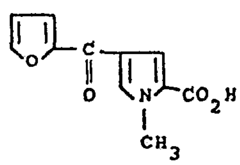 | 214 | F = 132 |
| 27 | 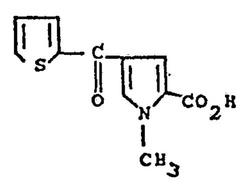 | 210 | F = 145 |
| 28 | 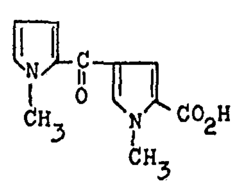 | 225 | F = 92 |

L'étude pharmacologique des composés de formule I a montré qu'ils ont une activité uricosurique et peuvent de ce fait être utilisés dans le traitement des hyperuricémies pathologiques, comme dans la goutte, ou pour éviter une augmentation de l'acide urique sanguin, à la suite de la prise de certains médicaments.

Cette activité uricosurique a été mise en évidence chez le rat. Elle se manifeste dès 50 mg/kg pour les composés de l'invention et pour certains dès 5 mg/kg, c'est-à-dire à une dose plus de 20 fois inférieure à la $DL_{50}$. Le test utilisé est celui décrit dans "J. Med. Pharm. Chem. *5* 175 (1962)".

Ce test n'est pas un dosage de l'acide urique, impossible chez les rongeurs mais une étude de la vitesse d'élimination du sang des rats d'un composé connu, le rouge phénol, après son administration aux animaux par voie I.V. On sait que lorsque les rats ont reçu des composés connus comme uricosuriques, tels que l'éthyl-2 benzofuranyl-3 hydroxy-4 diiodo-3,5 phényl cétone, l'acide (dipropylsulfamoyl-4) benzoïque ou la diphényl-1,2 (phénylsulfinyl-2) éthyl-4 pyrazolidine-3,5 dione, et ce préalablement à l'administration de rouge phénol, la vitesse d'élimination de ce colorant est diminuée.

Dans ces essais, les composés des exemples

ont des activités comparables à celles des produits précédement cités, mais à des doses jusqu'à 20 fois plus faibles.

La présente invention a donc également pour objet des compositions thérapeutiques contenant à titre de principe actif un composé de formule I, l'un de ses sels avec une base pharmaceutiquement acceptable ou l'un de ses dérivés fonctionnels se transformant par voie métabolique en ce composé, notamment en mélange avec un excipient pharmaceutiquement acceptable.

Les compositions thérapeutiques selon l'invention peuvent être administrées à l'homme par voie orale ou parentérale.

Ces compositions peuvent être notamment sous forme de gélules, comprimés, dragées, suspension ou solution injectable.

Ces compositions peuvent contenir notamment de 1 à 60% en poids de principe actif selon le mode d'administration.

La dose journalière par voie orale chez l'adulte peut être de 20 à 200 mg de principe actif.

**Revendications**

1. Composés de formule générale

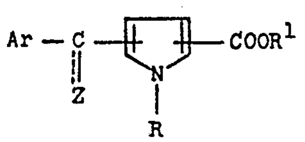

(I)

dans laquelle

$R^1$ représente un atome d'hydrogène ou un groupe alkyle ayant de 1 à 4 atomes de carbone,

R représente un groupe alkyle ayant de 1 à 6 atomes de carbone, un groupe benzyle ou un groupe phényle,

Ar représente un cycle aromatique choisi parmi les groupes phényle substitués ou non, les groupes naphtyle substitués ou non et les radicaux hétérocycliques aromatiques tels que thiényle, furyle ou pyrrolyle, et

>C⩦Z représente un groupe carbonyle >C=O, un groupe de formule >C=NOH ou un groupe alcoolique >CHOH, ainsi que les sels des acides de formule I avec des bases physiologiquement acceptables, et les dérivés fontionnels des composés de formule I qui se transforment par voie métabolique en ces composés.

2. Composés selon la revendication 1, caractérisés en ce que $R^1$ représente un atome d'hydrogène.

3. Composés selon la revendication 2, caractérisés en ce que R représente un groupe alcoyle ayant de 1 à 6 atomes de carbone.

4. L'acide méthyl-1(chloro-3 benzoyl)-4 pyrrole-2 carboxylique, son oxime, ses sels avec des bases physioligiquement acceptables et ses esters alcoyliques en $C_1$ à $C_4$.

5. L'acide méthyl-1(naphtoyl-1)-4 pyrrole-2 carboxylique, son oxime, ses sels avec des bases physiologiquement acceptables et ses esters alcoyliques en $C_1$ à $C_4$.

6. Composition thérapeutique ayant notamment une activité uricosurique, caractérisés en ce qu'elle comprend à titre de principe actif un composé selon l'une quelconque des revendications 1 à 5.

7. Procédé de préparation d'un composé de formule I telle que définie à la revendication 1, caractérisés en ce que l'on fait réagir un composé de formule II

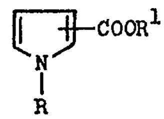

(II)

dans laquelle R et $R^1$ ont la signification donnée à la revendication 1 avec un acide de formule ArCOOH ou un dérivé réactif de celui-ci, cette réaction étant suivie, si cela est nécessaire, d'une réaction avec l'hydroxylamine en présence d'une base, d'une réduction de la fonction cétone et/ou d'une hydrolyse de l'ester carboxylique.

**Claims**

1. Compounds having the formula

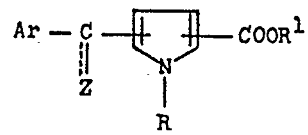

(I)

in which:

$R^1$ represents hydrogen or an alkyl group having 1 to 4 carbon atoms,

R represents an alkyl group having 1 to 6 carbon atoms, a benzyl group or a phenyl group,

Ar represents an aromatic ring selected from the substituted or unsubstituted phenyl groups, the substituted or unsubstituted naphthyl groups or the aromatic heterocyclic radicals such as thienyl, furyl or pyrrolyl and

>C⩦Z represents a carbonyl group >C=O, a group of the formula >C=NOH or an alcoholic group >CHOH

and the salts of acids of formula (I) with physiologically acceptable bases, and the functional derivatives of the compounds of the formula I which are metabolically transformed to these compounds.

2. Compounds as claimed in claim 1, wherein $R^1$ represents a hydrogen atom.

3. Compounds as claimed in claim 1 and 2, wherein R represents an alkyl group having 1 to 6 carbon atoms.

4. 1-Methyl 4-(3-chlorobenzoyl)pyrrole-2 carboxylic acid, its oxime and their salts with physiologically acceptable bases and their $C_{1-4}$ alkyl esters.

5. 1-Methyl 4-(1-naphtoyl)pyrrole-2 carboxylic acid, its oxime and their salts with physiologically acceptable bases and their $C_{1-4}$ alkyl esters.

6. A therapeutic composition having in particular an urocosuric acitivity characterized in that it comprises, as active ingredient, a compound as claimed in any one of the claims 1—5.

7. Process for the preparation of compounds of the formula (I) as defined in claim 1, characteized in that a compound of the formula (II)

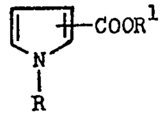

(II)

in which R and $R^1$ have the meanings defined in claim 1 is reacted with an acid of the formula ArCOOH or a reactive derivative thereof, this reaction being followed if necessary by a reaction with hydroxylamine in the presence of a base, a reduction of the ketone function and/or a hydrolysis of the carboxylic ester.

**Patentansprüche**

1. Verbindungen der allgemeinen Formel I

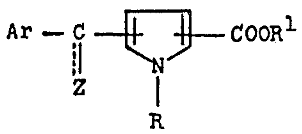

in der $R^1$ für Wasserstoff oder eine Alkylgruppe mit 1—4 Kohlenstoffatomen, R für eine Alkylgruppe mit 1—6 Kohlenstoffatomen, eine Benzylgruppe oder eine Phenylgruppe, Ar für einen aromatischen Ring, wie substituierte oder nicht-substituierte Phenylgruppen, substituierte oder nicht-substituierte Naphthylgruppen, heterozyklische aromatische Gruppen wie Thienylgruppen; Furyl- oder Pyrrolygruppen und >C=Z für eine Carbonylgruppe >C=O, eine Gruppe der Formel >C=NOH oder eine Alkoholgruppe >CHOH, stehen, sowie die Salze der Säuren gemäß Formel I mit physiologisch verträglichen Basen, sowie die funktionellen Derivate der Verbindungen gemäß Formel I, die sich metabolisch in jene Verbindungen umwandeln.

2. Verbindungen gemäß Anspruch 1, dadurch gekennzeichnet, daß $R^1$ für Wasserstoff steht.

3. Verbindungen nach Anspruch 2, dadurch gekennzeichnet, daß R für eine Alkylgruppe mit 1—6 Kohlenstoffatomen steht.

4. 1-Methyl-4 (3-chlor-benzoyl)-2-pyrrolcarbonsäure deren Oxime, deren Salze mit physioligisch verträglichen Basen und deren Alkylester mit 1—4 Kohlenstoffatomen.

5. 1-Methyl-4 (naphthoyl)-2-pyrrol-carbonsäure, deren Oxime, deren Salze mit physiologisch verträglichen Basen und deren Alkylester mit 1—4 Kohlenstoffatomen.

6. Therapheutische Zusammensetzung, insbesondere mit Wirkung als Uricosurica, gekennzeichnet durch Gehalt an einer Verbindung gemäß einem der Ansprüche 1—5 als Wirkstoffkomponente.

7. Verfahren zur Herstellung einer Verbindung der Formel I gemäß Anspruch 1, dadurchgekennzeichnet, daß man eine Verbindung der Formel II

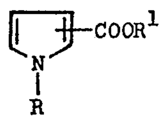

in der R und $R^1$ gemäß Anspruch 1 definiert sind, mit einer Säure der Formel ArCOOH oder einem reaktiven Derivat dieser Säure umsetzt, worauf man, falls nötig, mit Hydroxylamin in Gegenwart einer Base umsetzt, eine Reduktion der Ketogruppe und/oder eine Hydrolyse des Carbonsäureesters durchführt.